# EUROPEAN PATENT SPECIFICATION

(11) **EP 4 139 324 B1**
(45) Date of publication and mention of the grant of the patent: **15.10.2025**
(21) Application number: 21719150.1
(22) Date of filing: 19.04.2021
(51) Int. Cl.: C07J 9/00, C07J 41/00

(54) **ORGANIC SOLVENT NANOFILTRATION OF 7-DEHYDROCHOLESTEROL OR 25-HYDROXY-7-DEHYDROCHOLESTEROL OR THEIR OH PROTECTED FORMS**
ORGANISCHE LÖSUNGSMITTEL-NANOFILTRATION VON 7-DEHYDROCHOLESTERIN ODER OH-GESCHÜTZTEN FORMEN DAVON
NANOFILTRATION DE SOLVANT ORGANIQUE DE 7-DÉHYDROCHOLESTÉROL OU SES FORMES PROTÉGÉES OH

(30) Priority: 23.04.2020 EP 20171013
(43) Date of publication of application: 01.03.2023
(73) Proprietor: DSM IP Assets B.V., 6221 BE Maastricht (NL)
(72) Inventor: GOY, Roman, 4303 Kaiseraugst (CH); MARTY, Maurus, 4303 Kaiseraugst (CH); SCHUETZ, Jan, 4303 Kaiseraugst (CH); WAECHTER, Ralph, 4303 Kaiseraugst (CH); WITTE, Julia, 4303 Kaiseraugst (CH)
(74) Representative: DSM IP ANH
(86) International application number: PCT/EP2021/060109
(87) International publication number: WO 2021/213988

(56) References cited:
- CN-A- 101 381 389
- E V YABLONSKAYA ET AL: "Synthesis of 7-dehydrocholesterol acetate", CHEMISTRY OF NATURAL COMPOUNDS, vol. 9, 1 January 1973 (1973-01-01), pages 708 - 709, XP055737754
- ISSARA SEREEWATTHANAWUT ET AL: "Demonstration of Molecular Purification in Polar Aprotic Solvents by Organic Solvent Nanofiltration", ORGANIC PROCESS RESEARCH & DEVELOPMENT, vol. 14, no. 3, 21 May 2010 (2010-05-21), US, pages 600 - 611, XP055738244, ISSN: 1083-6160, DOI: 10.1021/op100028p

## Description

### Technical Field

The present invention relates to the purification of 7-dehydrocholesterol or 25-hydroxy-7-dehydrocholesterol or their OH-protected forms, respectively.

### Background of the invention

7-Dehydrocholesterol and 25-hydroxy-7-dehydrocholesterol are key intermediate used in the synthesis for cholecalciferol (=vitamin D3).

Nanofiltration is a relatively new unit operation of fluid processing. Organic Solvent Nanofiltration (OSN) is a membrane-based separation process on a molecular level. Details of the technique and the applications used in nanofiltration are disclosed for example by C. Schnitzer et al., Chem. Ing. Tech. 2014, 86(5), 589-593 and M. Priske et al., Chem. Ing. Tech. 2016, 88(1), 39-49 as well as by P. Marchetti et al., Chem Rev. 2014, 114, 10735-10806.

C. Allègre et al., J. Membrane Science 2006, 269, 109-117 discloses the separation of cholesterol and glycerophospholipids in ethanol by nanofiltration. However, these two ingredients have not only different molecular weight but also strongly different structures.

Chemistry of Natural Compounds Vol 9, pp 708-709 (1973) and CN-A-101 381 389 both disclose a hydrazone-based synthesis of 7-dehydrocholesterol and the former citation also discloses its purification by crystallisation from methanol.

It has been observed that a new process of synthesis of 7-dehydrocholesterol, or its OH-protected form, yield a mixture of of 7-dehydrocholesterol, or its OH-protected form, and a specific higher molecular species, unknown until today, which are difficult to separate from each other by known separation techniques.

### Summary of the invention

Therefore, the problem to be solved by the present invention is to provide an efficient method of purification of 7-dehydrocholesterol or 25-hydroxy-7-dehydrocholesterol or their OH-protected form from a mixture of 7-dehydrocholesterol or 25-hydroxy-7-dehydrocholesterol or their OH-protected form and a specific and new higher molecular compound of formula (V) formed in a new process of synthesis.

The process according to claim 1 is able to solve this problem.

The compound of formula (I) has been identified and its structure characterized. This compound of formula (V) is coloured and can be used as a new colourant, however, this compound of formula (V) does not form part of the present invention.

Said method yields a separation of the new compound of formula (V) and 7-dehydrocholesterol, or 25-hydroxy-7-dehydrocholesterol or their OH-protected form, allowing to use both molecules for separate purposes. 7-Dehydrocholesterol, or 25-hydroxy-7-dehydrocholesterol or their OH-protected forms can be used for the synthesis of vitamin D3 using known photochemical transformation reactions. The compound of formula (V) can be used as colorant in diverse applications, however, this compound of formula (V) does not form part of the present invention.

Further aspects of the invention are subject of further independent claims. Particularly preferred embodiments are subject of dependent claims.

### Detailed description of the invention

In a first aspect the present invention relates to a process of purification of a compound of the formula (I) comprising the subsequent steps
a) providing a solution of a compound of formula (I) and a compound of formula (V) in a suitable solvent 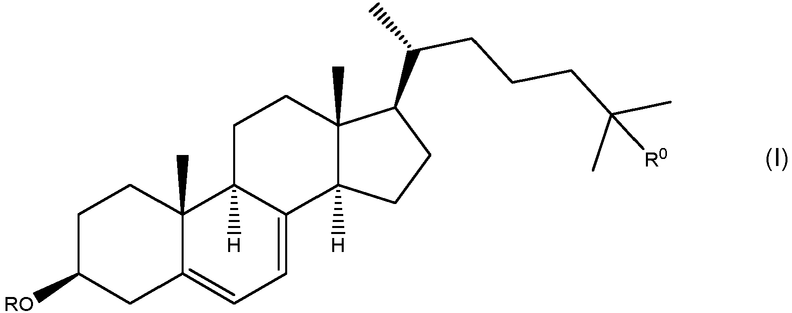 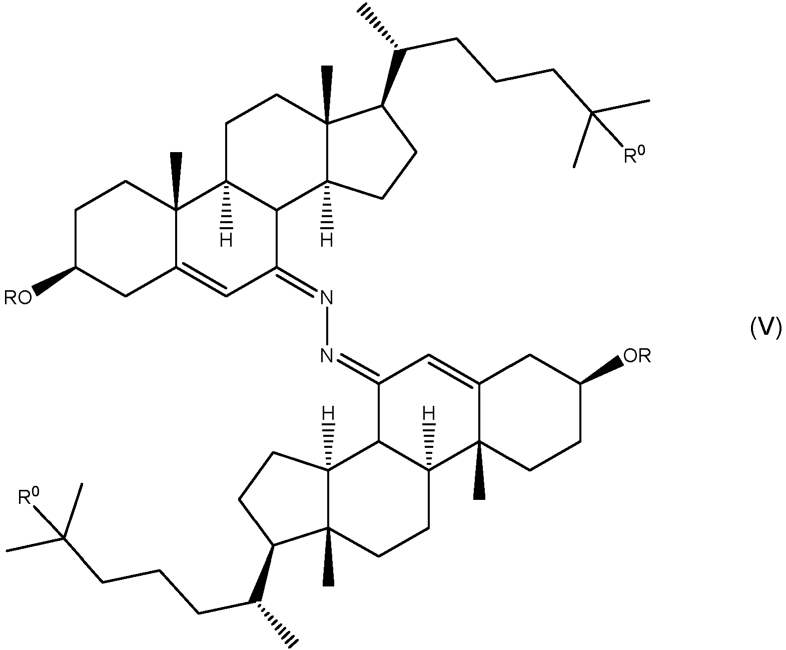
b) subjecting the solution of step a) by organic solvent nanofiltration using a membrane;
c) isolating the compound of formula (I) from the permeate obtained from step b)
wherein R represents H or an OH-protecting group; and R⁰ represents H or OH or OR' wherein R' represents an OH-protecting group.

For sake of clarity, some terms as used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group. Hence, "propyl" can be "*n*-propyl" or "*iso*-propyl" (=isopropyl). Analogously, "butyl" can be "*n*-butyl" or "*iso*-butyl" or "*sec*-butyl" or "*tert*-butyl".

In the present document, "aralkyl" group is an alkyl group of which at least one H is substituted by an aryl group. Hence, for example, benzyl (=C₆H₅-CH₂-) is a C₇-aralkyl group.

In the present document, "alkylaryl" group is an aryl group of which at least one H of the aromatic ring is substituted by an alkyl group. Hence, for example, ethylphenyl (CH₃-CH₂-C₆H₄-) is a C₈-alkylaryl group.

In case identical labels for symbols or groups are present in several formulae, in the present document, the definition of said group or symbol made in the context of one specific formula applies also to other formulae which comprises the same said label.

The "molecular weight cut-off" describes the retention capability of a membrane and refers to the molecular weight of a solute with a membrane retention of 90%. The molecular cut-off for a specific commercial membrane is typically determined by use of specific reference molecules having specific molecular weights (MW). For the indication of the molecular weight cut-off used in this document said reference molecules are tetracosane (MW=339 g/mol) in toluene, hexatriocontane (MW=507 g/mol) in toluene, polystyrene (MW=580 g/mol) in heptane and polystyrene (MW=1300 g/mol) in heptane.

R represents H or an OH-protecting group. R⁰ represents H or OH or OR' wherein R' represents an OH-protecting group. An OH-protecting group is a group which protects a hydroxyl group and the protecting group can be easily removed, i.e. by state-of-the-art methods, resulting to the respective compound with the free alcoholic group again.

The OH-protecting group is introduced by a chemical reaction of the compound of the respective formula having H instead of the OH-protecting group with a protecting agent.

The protecting agents leading to the corresponding OH-protecting groups are known to the person skilled in the art, as well as the chemical process and conditions for this reaction. If, for example, the OH-protecting group forms with the rest of the molecule an ester, the suitable protecting agent is for example an acid, an anhydride, or an acyl halide.

The OH-protecting group is particularly selected from the groups consisting of 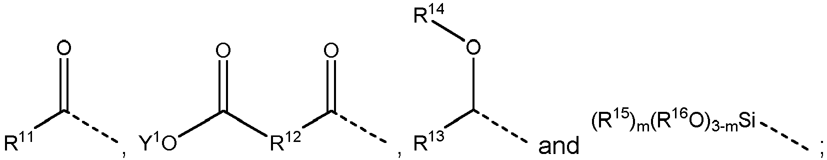
wherein R¹¹ represents H, a C₁₋₁₅-alkyl or a fluorinated C₁₋₁₅-alkyl or a C₁₋₁₅-cycloalkyl or a C₇₋₁₅-aralkyl group or a C₇₋₁₀-arylalkyl group or a phenyl group;
R¹² represents a C₁₋₁₅-alkylene or a C₆₋₁₅-alkylene group;
and wherein either
   R¹³ represents a C₁₋₁₅-alkyl group or an alkyleneoxyalkyl group or a polyoxyalkylene group;
   R¹⁴ represents hydrogen or a C₁₋₁₅-alkyl group;
      or
   R¹³ and R¹⁴ represent together a C₃₋₇-alkylene group forming a 5 to 7 membered ring;
and wherein R¹⁵ represents a C₁₋₁₅-alkyl group and R¹⁶ represents a C₁₋₄-alkyl group and m = 0 or 1 or 2 or 3, preferably m=3;
and wherein the single dotted line represents the bond by which said substituent is bound to the rest of a molecule.
If R and/or R' is represented by 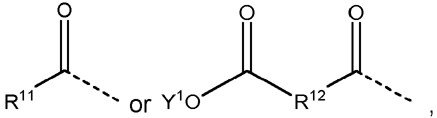 the respective compound is an ester of a carboxylic acid or dicarboxylic acid, which can be formed by the reaction of the respective protecting agent with the hydroxyl group. In this case, the protecting agent may be for example an anhydride or halide of the respective carboxylic acid (1) or dicarboxylic acid (2).

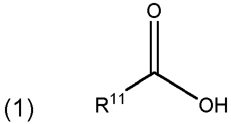

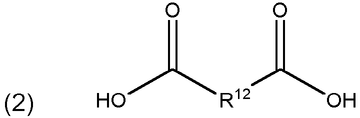

If the compound of the respective formula is an ester of a carboxylic acid or dicarboxylic acid, it is preferred that R and/or R' is an C₁₋₇-acyl, preferably acetyl, trifluoroacetyl, propionyl or benzoyl group, or a substituted benzoyl group.

Esters can be easily deprotected under the influence of an acid or a base.

If R and/or R' is 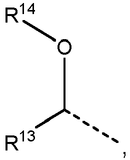 the respective compound is an acetal, which can be formed by the reaction of the respective protecting agent with the hydroxyl group. In this case, the protecting agent may be for example, a respective aldehyde, alkyl halide, e.g. MeO(CH₂)₂OCH₂Cl, or an enol ether, e.g. 3,4-dihydro-2*H*-pyran.

In this case, the substituent R and/or R' is preferably 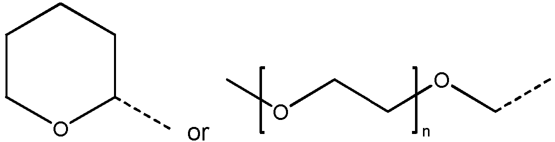 with n=0 or 1.

In some instances, acetals are also called "ethers", particularly in the cases mentioned above: methoxymethyl ether (MOM-ether), β-methoxyethoxymethyl ether (MEM-ether) or tetrahydropyranyl ether (THP-ether).

Acetals can be easily deprotected under the influence of acids.

In another preferred embodiment, the respective compound is an ester of phosphoric acid, pyrophosphoric acid, phosphorous acid, sulphuric acid or sulphurous acid.

Depending on the reaction conditions, the esterification is either complete or partial, leaving some residual acid groups of the respective acid non-esterified.

It is most preferred that the protecting group R is a benzoyl group or a C₁₋₄-acyl group, particularly acetyl or trifluoroacetyl group, more particularly acetyl group. The molecules in which R and/or R' represents an acyl group, particularly an acetyl group, can be easily prepared from the corresponding unprotected molecule by esterification, and the unprotected alcohol can be obtained from the corresponding ester by ester hydrolysis, particular by strong bases such as NaOH or KOH.

It is preferred that the protecting group R and/or R' represents an acyl group, preferably an acetyl or benzoyl group, particularly an acetyl group:

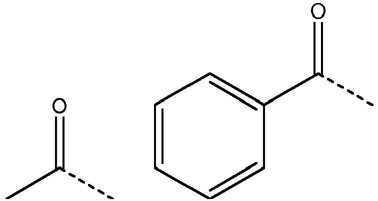

In one embodiment R and/or R' represents an OH-protecting group.

In the other embodiment R represents H.

In another embodiment R⁰ represents OH.

In a further embodiment R⁰ represents H.

In a preferred embodiment R represents H and R⁰ represents OH.

In a most preferred embodiment R represents H and R⁰ represents H.

In step a) a solution of a compound of formula (I) and a compound of formula (V) in a suitable solvent is provided.

Compound of formula (I) is 7-dehydrocholesterol (DHC) or 25-hydroxy-7-dehydrocholesterol (HyDHC) or is protected derivative. Preferably the compound of formula (I) is 7-dehydrocholesterol, i.e. R⁰ is preferably H. It has been observed that a mixture of compound of formula (I) and of formula (V) are obtained by a new synthesis of compound of formula (I).

Hence, a mixture of compound of formula (I) and of formula (V) can be obtained when a salt of the formula (II) is added to a solvent of the formula (III) at a temperature of between 60 °C and the boiling point of said solvent 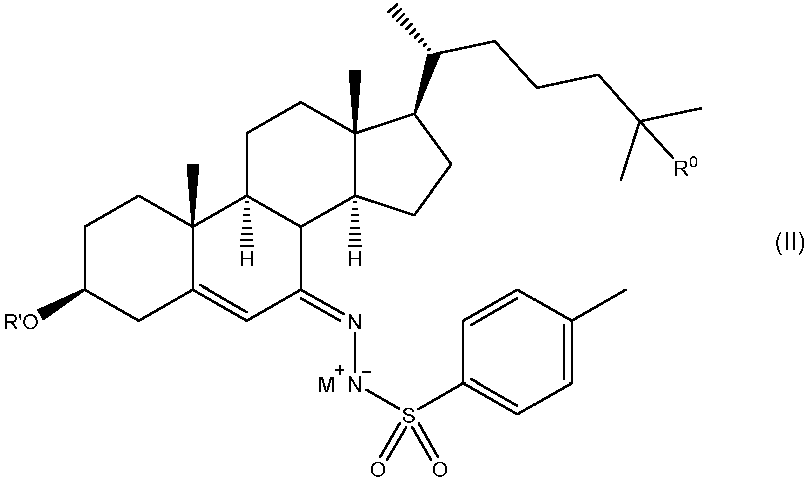 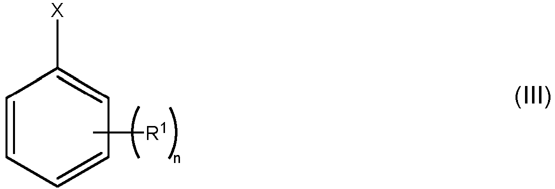 characterized in that
R' represents an OH-protecting group;
M is an alkali metal atom, preferably Li;
X represents either a halogen atom, preferably Cl, or a C₁₋₆-alkoxy group;
R¹ represents an C₁₋₆-alkyl group;
n = 0 or 1 or 2 or 3, preferably 0;
with the proviso, that if R represents H, the protecting group R' needs to be deprotected to yield the free OH group.

It is preferred that the compound of the formula (II) is prepared by salt formation of a compound of the formula (IV) 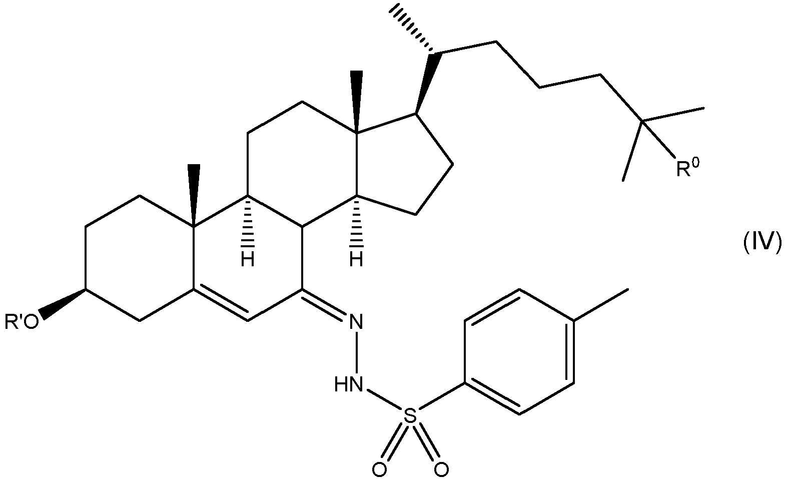 and a strong base comprising an alkali metal, particularly an alkali metal alcoholate, amide, hydride or complex hydride or a alkali metal alkyl, particularly butyllithium, preferably by NaNH₂ or LiNH₂.

The compound of formula (IV) is readily available from tosylhydrazide and the compound of formula(IV-a) which itself can be obtained from compound (IV-b):

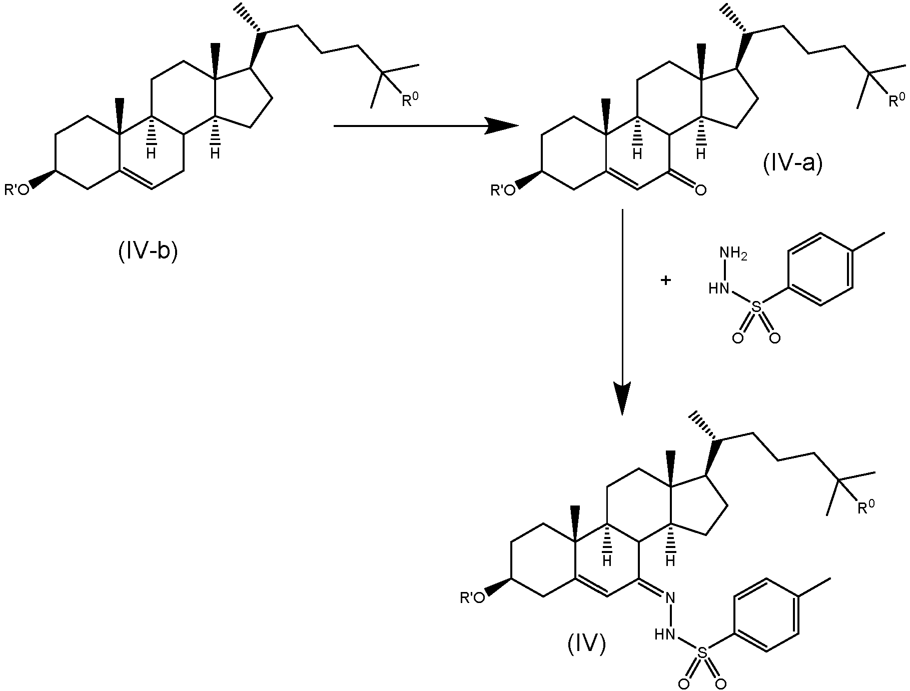

The alkali metal amide is preferably obtained by in situ formation from a mixture of the respective alkali metal and ammonia, particularly in the presence of an iron catalyst.

The formation of a salt of the formula (II) is preferably made in a solvent of formula(III).

Preferably, the base is suspended in the solvent of formula (III) is mixed with compound of formula (IV) at a temperature preferably of lower than 50°C, preferably between 10 and 45°C, most preferably at around room temperature, forming the salt in a dispersion.

It is preferred that the amount of solvent of formula (III) in this dispersion is less than 35%, preferably less than 30 % of the solvent of formula (III) used in the adding step.

Principally, the solvent can be removed and the salt can be isolated and added in the adding step as solid salt or the salt can be added as said dispersion. The base is preferably used in a stoichiometric excess relative to compound of formula (IV). Typically the molar ratio of base/compound of formula (IV) is between 1.5: 1 and 10:1, more preferably between 2:1 and 4:1.

In the adding step the solvent of formula (III) is heated to a temperature of between 60 °C and the boiling point of said solvent. To said warm solvent the salt of formula (II) is then added, preferably under stirring. It has been found that it is preferably that the salt is added slowly.

Hence, the salt of formula (II) is preferably added over a time range of between 15 and 60 minutes, preferably between 25 and 40 minutes.

This new synthesis of process 7-dehydrocholesterol or 25-hydroxy-7-dehydrocholesterol, or their OH-protected form, yields particularly mixtures in the ratio ***w_{V}**l**w_{I}*** of between 0.00001% and 0.7%, preferably between 0.00001% and 0.6%, particularly between 0.00001% and 0.5%, wherein ***w_{I}*** is the weight of the compound of the formula (I) and ***w_{V}*** is the weight of the compound of the formula (V).

In step a) a solution of compound of formula (I) and (V) is formed using a suitable organic solvent. Suitable solvents are those solvents in which the compounds of formula (I) and (V) are dissolved at a temperature of between 10°C and 100°C, particularly between 10 and 60°C, so that a homogenous solution without any solid particles is formed. The amount of compounds of formula (I) and (V) dissolvable depends strongly on the solvent used.

It is preferred that the concentration of the compound of formula (I) in the solution at room temperature of step a) is between 0.1 and 20 % by weight, preferably between 0.5 and 10 % by weight, relative to the weight of the reaction mixture.

It is further preferred that in that the weight ratio of the compound of formula (V) to the compound of formula (I) in the solution of step a) is between 1:1'000'000 and 1:50, preferably between 1:900'000 and 1:200.

In one embodiment, the suitable solvent is preferably a mixture of at least one alcohol and at least one C₅₋₁₀-alkane, preferably a mixture of a C₁₋₆-alcohol and a C₅₋₁₀-alkane, most preferably a mixture of hexane or heptane and methanol or ethanol, mostly preferred a mixture of hexane and methanol.

For the embodiments of the compound of the formula (I) being 25-hydroxy-7-dehydrocholesterol or its protected form (i.e. R⁰≠H), the suitable solvent is preferably either tetrahydrofuran or cyclopentylmethyl ether or isopropanol or a mixture of at least one alcohol and at least one C₅₋₁₀-alkane, preferably a mixture of a C₁₋₆-alcohol and a C₅₋₁₀-alkane, most preferably a mixture of hexane or heptane and isopropanol or methanol or ethanol, mostly preferred a mixture of hexane and isopropanol.

In another embodiment, the solvent used is preferably of formula (III) 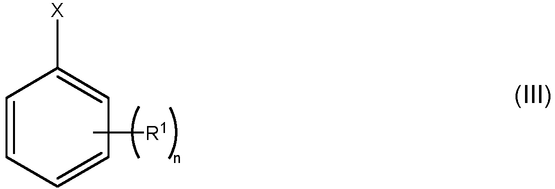 wherein
X represents either a halogen atom, preferably Cl, or a C₁₋₆-alkoxy group;
R¹ represents an C₁₋₆-alkyl group; and
n = 0 or 1 or 2 or 3, preferably 0;
preferably chlorobenzene.

In a first variant, X represents C₁₋₆-alkoxy group. The methoxy group A is the preferred C₁₋₆ alkoxy group.

In this variant, the solvent of formula (III) is preferably selected from the group consisting of anisole, 2-methylanisiole, 3-methylanisole, and 4-methylanisole, preferably anisole.

In another, preferred, variant X represents a halogen atom, preferably Cl.

Hence, compound of formula (III) can have up to 3 C₁₋₆-alkyl groups bound directly on the aromatic ring. The solvent of formula (III) is preferably selected from the group consisting of chlorobenzene, o-chlorotoluene, m-chlorotoluene, p-chlorotoluene. Most preferred, the solvent of formula (III) is chlorobenzene.

It is preferred that that the weight ratio of alcohol to C₅₋₁₀-alkane is between 2:98 and 75:25, preferably between 10:90 and 65:35, most preferably between 15:85 and 50:50.

In step b) the solution of step a) is subjected to organic solvent nanofiltration using a membrane.

The permeate passes easily through the membrane whereas the retentate is not, or at least significantly less, able to pass the membrane. The higher molecular species are part of the retentate whereas the lower molecular species are part of the permeate.

There are different membranes available which have a respective molecular cut-off properties of different materials, inorganic or organic.

It is of course substantial that the membrane needs to be stable against the solvents in use.

However, it has been shown that particularly suitable membranes are polymeric membranes, preferably membranes based on silicone, most preferably membranes which are silicone-based composites. Most preferred are membranes based on PDMS/PAN (polydimethylsiloxane/polyacrylonitrile).

As the molecular weight cut-off is an important property of the membrane used for the separation of the lower and higher molecular species it is preferred for the present invention to use a membrane which has a molecular weight cut-off MWCO) of between 250 and 1000 Dalton, preferably of between 300 and 400 Dalton.

Particularly suitable are membranes which are commercially available for example as *oNF-1* (MWCO = ca. 600 Dalton), *oNF-2* (MWCO = ca. 350 Dalton) or *oNF-3* (MWCO = ca. 900 Dalton) from Borsig Membrane Technology GmbH, Germany.

It is preferred that the pressure applied in the organic solvent nanofiltration of step b) is between 0.1 and 60 bar, preferably between 0.5 and 50 bar, more preferably 5 and 50 bar, even more preferably between 1 and 20 bar, most preferably between 8 and 15 bar.

If the pressure is too high, the passing is reduced as the high pressure tends to decrease the size of the pores of the membrane.

It is further preferred that the organic solvent nanofiltration of step b) is performed at a temperature of between 15 and 70, preferably between 15 and 60°C, more preferably between 20 and 50°C.

The equipment used in organic solvent nanofiltration is known to the person skilled in the art.

It has been shown that the organic solvent nanofiltration is particularly preferred to be performed in a cross flow mode.

In the cross-flow mode the feed flows tangentially over the membrane surface and perpendicularly to the permeate flux.

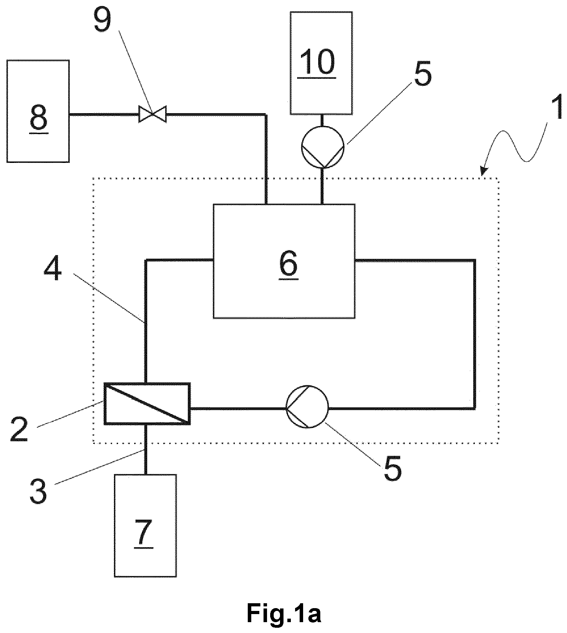

It is preferred to use the nanofiltration in a cross-flow mode, in which the retentate is refeed into the inlet of the filtration unit as shown schematically in figure 1a.

The solution of compound of formula (I) and of formula (V) is pumped from the vessel of said solution (10) to the reservoir (6) of the organic solvent nanofiltration unit (1) by means of a pump. The reservoir (6) is pressurized by nitrogen (8) to the desired pressure by means of a gas pressure regulator (9). The reservoir (6) is heated so as to provide and to maintain a desired temperature for the solution. The solution in the reservoir is pumped by means of a pump (5) onto the OSN membrane (2) which separates the permeate (3) form the retentate (4). The OSN membrane (2) is preferably arranged in a cross-flow mode. Said retentate (4) is continuously pumped over the OSN membrane (2) for a specific time. The permeate (3) is collected in a collection vessel for permeate (7). In the permeate (3) a significantly higher weight ratio of compound of formula (I) / compound of formula (V) is observed as compared to the solution provided in the vessel (10). In the retentate (4), on the other hand, a significantly lower weight ratio of compound of formula (I) / compound of formula (V) is observed as compared to the solution provided in the vessel (10).

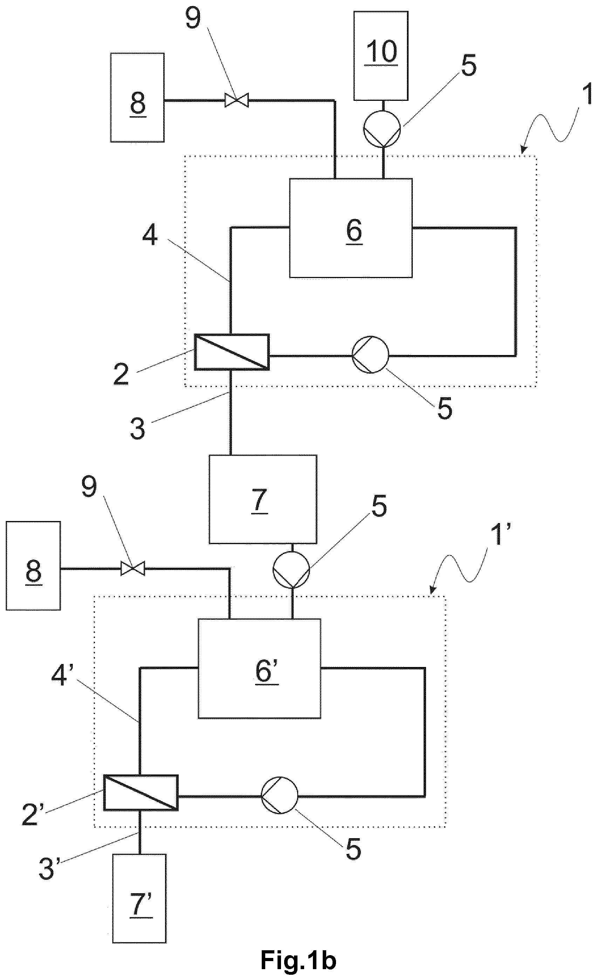

In a particularly preferred manner, the filtrations are performed in a multi-stage nanofiltration as shown schematically in figure 1b.

The solution of compound of formula (I) and of formula (V) is pumped from the vessel of said solution (10) to the reservoir (6) of a first organic solvent nanofiltration unit (1) by means of a pump. The reservoir (6) is pressurized by nitrogen (8) to the desired pressure by means of a gas pressure regulator (9). The reservoir (6) is heated so as to provide and maintain a desired temperature for the solution. The solution in the reservoir is pumped by means of a pump (5) onto the OSN membrane (2) which separates the permeate (3) form the retentate (4). The OSN membrane (2) is preferably arranged in a cross-flow mode. Said retentate (4) is continuously pumped over the OSN membrane (2) for a selected time. The permeate (3) is collected in a collection vessel for permeate (7).

Said vessel for permeate (7) of the first organic solvent nanofiltration unit (1) serves as a reservoir from which said permeate (3) is pumped into the reservoir (6') of a second organic solvent nanofiltration unit (1'). The reservoir (6') is pressurized by nitrogen (8) to the desired pressure by means of a gas pressure regulator (9). The reservoir (6) is heated so as to provide and maintain a desired temperature for the solution. The solution in the reservoir is pumped by means of a pump (5) onto the OSN membrane (2'). Said OSN membrane (2') has a different molecular weight cut-off as the OSN membrane (2) from the first organic solvent nanofiltration unit (1). Preferably the OSN membrane (2') of the second organic solvent nanofiltration unit (2') has a lower molecular weight cut-off as the OSN membrane (2) from the first organic solvent nanofiltration unit (1). More preferably, the OSN membrane (2') of the second organic solvent nanofiltration unit (2') has a molecular weight cut-off of about 350 Dalton and the OSN membrane (2) from the first organic solvent nanofiltration unit (1) has a molecular weight cut-off of about 900 Dalton.

Said OSN membrane (2') separates the permeate (3') form the retentate (4'). The OSN membrane (2') is preferably arranged in a cross-flow mode. Said retentate (4') is continuously pumped over the OSN membrane (2') for a specific time.

The permeate (3') is collected in a collection vessel for permeate (7').

In the permeate (3) a significantly higher weight ratio of compound of formula (I) / compound of formula (V) is observed as compared to the solution provided in the vessel (10). In the retentate, on the other hand, a significantly lower weight ratio of compound of formula (I) / compound of formula (V) is observed as compared to the solution provided in the vessel (10).

It is, furthermore, observed that in the permeate (3') of the second nanofiltration unit (1') a significantly higher weight ratio of compound of the formula (I) to compound of the formula (V) is present as compared to the permeate (3) leaving the first organic solvent nanofiltration unit (1) and collected in collection vessel (7). In the retentate (4'), on the other hand, a significantly lower weight ratio of compound of formula (I) / compound of formula (V) is observed as compared to the permeate (3) leaving the first organic solvent nanofiltration unit (1) and collected in collection vessel (7).

It can be advantageous to combine more than two nanofiltration units in sequence in analogy as disclosed above, particular by figure 1b, for two nanofiltration units to provide a multi-stage nanofiltration.

To increase the separation capacity, nanofiltration units can be also combined in parallel using the same OSN membrane. It is, of course, further possible to increase separation capacity by increasing the size (membrane surface) of the OSN membrane in an individual OSN nanofiltration unit.

This allows nanofiltration of solutions of compounds of formula (I) and (V) in large, particular industrial scale.

For the use in industrial scale, it is preferred that the OSN membrane is in form of spiral wound elements (SWE).

Surprisingly, it has been observed that using a two stage or multi-stage nanofiltration (according to figure 1b) a significantly better separation of compounds of formula (I) and (V) can be achieved that using an organic solvent nanofiltration with only one OSN membrane (according to figure 1a).

Hence, it is preferred that the nanofiltration is a two-stage nanofiltration (figure 1b)) combining the use of two individual nanofiltration units having membranes of different molecular cut-off in sequence. In this two-stage nanofiltration it is preferred that the membrane (2) used in the first stage (i.e. first nanofiltration unit(1)) has a higher MWCO than the membrane (2') used in the second stage (i.e. second nanofiltration unit (1')).

The separation of compounds of formula (I) and (V) by the described process is to significant degree.

It is possible that by said purification process that 7-dehydrocholesterol or 25-hydroxy-7-dehydrocholesterol is obtained which has an amount of less than 300 ppm, preferably less than 200 ppm, more preferably less than 100 ppm, most preferably less than 50 ppm, of compound of formula (V).

As discussed above a mixture of compound of formula (I) and compound of formula (V) can be obtained when a salt of the formula (II) is added to a solvent of the formula (III) at a temperature of between 60 °C and the boiling point of said solvent.

Compound of formula (V) has a significantly higher molecular weight as compared to compound of formula (I).

Compound of formula (V) can be isolated, for example by extraction or crystallization or chromatography, particularly by HPLC, from the retentate obtained from the step b) of the process of purification as described above in great detail.

A particular easy way for isolation of the compound of formula (V) has been found when a mixture of hexane and methanol is used in step b) as the solubility of the compound of formula (V) is limited. When its concentration exceeds 3000 ppm, the compound of the formula (V) tends to precipitate. When adding a filtration aid to the retentate after collected from the nanofiltration unit at the end of the nanofiltration, the compound of formula (V) which is bound to the filtration aid can be easily filtered off and extracted to obtain the compound of formula (V).

Of course, depending on the solvent used, the retentate solution having not yet exceeded the solubility maximum of compound of formula (V) can be concentrated by evaporating said solvent to initiate the precipitation process of compound of formula (V).

The structure of the new compound of formula (V) has been fully identified and the compound is characterized particularly by ¹H-NMR, ¹³C-NMR and MS.

Compound of formula (V) (R=OH, R⁰=H):
¹H-NMR (300 MHz, CDCl₃): δ (ppm) = 0.72 (s, 6H), 0.86 (d, 6H, J=1.32 Hz), 0.88 (d, 6H, J=1.32 Hz), 0.94 (d, 6H, J=6.40 Hz), 1.01 - 1.63 (m, 42H), 1.80 (s, 2H), 1.90 (m, 6H), 2.04 (d, 2H, J = 12.62 Hz), 2.38 (m, 6H) 2.56 (m, 2H), 3.63 (m, 2H), 6.37 (s, 2H);
¹³C-NMR (75 MHz, CDCl₃): δ (ppm) = 12.3,17.9, 19.0, 21.0, 22.6, 22.9, 23.8, 24.6, 27.4, 28.0, 31.5, 35.7, 36.3, 36.6, 38.5, 38.6, 39.6, 39.8, 42.5, 43.0, 49.6, 50.5, 54.7, 71.2, 117.4, 153.5, 161.2.
LC-MS (ES) m/z: 797 [M⁺], 780 [M⁺+H-H₂O], 382 [monomer of M⁺+H-H₂O].

The compounds having protected groups (such as acetate) could been identified accordingly as well.

Whilst the compound of formula (V) and its isolation are disclosed herein, these do not form part of the present invention.

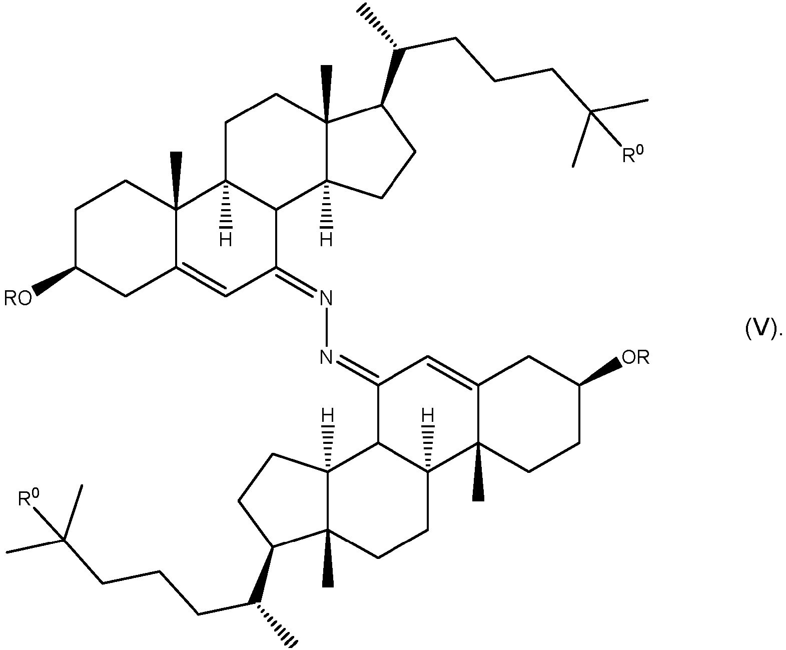

The residue R represents either H or an OH-protecting group.

The residue R⁰ represents either H or OH or OR', wherein R' represents an OH-protecting group.

The OH-protecting group is as described already before for the compound of formula (I).

The OH-protecting group is preferably an acyl group, preferably an acetyl group.

The compound of formula (V) is yellow and can be used as a new colorant, and is suitable particularly in the field of colouring polymers and food and feed.

As mentioned above the compound of formula (V) is formed at very low amounts parallel to the formation of compound (I).

It has been found that the above described process of purification of a compound of the formula (I) can be used for the purification of 7-dehydrocholesterol or 25-hydroxy-7-dehydrocholesterol or its resp. their OH-protected derivatives.

Hence, in a further aspect the present invention relates to the use of organic solvent nanofiltration for the purification of a composition comprising compound of formula (I) 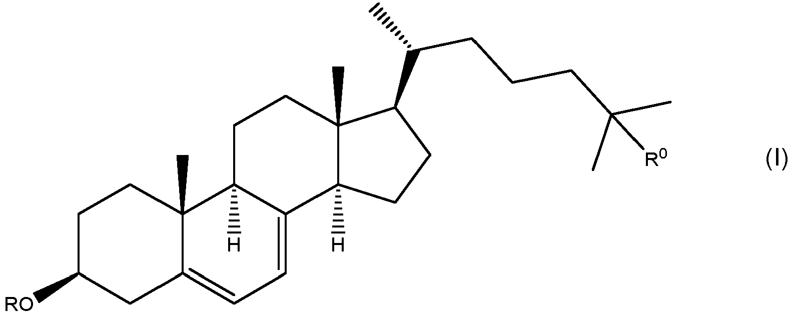
wherein R represents H or an OH-protecting group and R⁰ represents H or
OH or OR' wherein R' represents an OH-protecting group.

### List of reference signs

- 1,1': Organic Solvent Nanofiltration unit
- 2, 2': OSN membrane
- 3, 3': Permeate
- 4, 4': Retentate
- 5: Pump
- 6, 6': Reservoir
- 7,7': Collection vessel for permeate
- 8: Nitrogen container
- 9: Gas pressure regulator
- 10: Vessel for solution of compound of formula (I) and (V)Premixture

### Examples

The present invention is further illustrated by the following experiments.

### Preparation of solution of compound of formula (I) and (V) with R⁰=H

In a first stage the lithium salt of formula (II) has been prepared by 2.972 equivalents of LiNH₂ have been added to 20-30% of the total amount of chlorobenzene (for obtaining in the second stage a concentration of 7-tosylhydrazone cholesterylacetate in chlorobenzene of 0.09 g/ml) and stirred. Then 1.0 equivalent of 7-tosylhydrazone cholesterylacetate (=Compound of formula (IV), R'=CH₃COO) has been added at 23°C under stirring during 30 minutes, forming a dispersion of the Li salt (=compound of formula (II), M=Li, R'=CH₃COO) (***LiSalt***)*.*

In a second stage the rest (i.e. 70-80 %) of the total amount of chlorobenzene has been separately heated up to 130-135°C. The dispersion of the above Li salt (***LiSalt***) has been added under stirring for 30 minutes to said hot chlorobenzene. The reaction mixture was stirred for about 3-5 hours at a temperature of 130-135°C. Then the mixture has been filtered. The filtrate was extracted twice with water and once with saturated NaCl solution. After evaporation of the chlorobenzene the product has been isolated. The 7-dehydrocholesterol acetate has been saponified by KOH and subsequent neutralization using acetic acid to yield 7-dehydrocholesterol.

For the following experiments a mixture of compounds of 7-dehydrocholesterol (DHC) and compound of formula (V) (with R⁰=H) obtained by the above procedure has been dissolved in a solvent mixture consisting of hexane/methanol (69/31 w/w) to obtain a starting solution of 7.7 % DHC.

### Experimental series 1: One stage nanofiltration (according to figure 1a)

In the first series of examples, said starting solution has been objected during 4 hours in a crossflow mode to a nanofiltration using a membrane (***Memb.***) as indicated in table 1
oNF-1:MWCO = ca. 600 Dalton, Borsig Membrane Technology GmbH, Germany
oNF-2:MWCO = ca. 350 Dalton, Borsig Membrane Technology GmbH, Germany
*oNF-3:*MWCO = ca. 900 Dalton, Borsig Membrane Technology GmbH, Germany
having a surface area of 42 cm² arranged in an experimental layout as above described in figure 1a.

The pressure (***p***), the temperature (***T***) and the loop feed (***LF***) used for the experiments have been indicated in table 1.

From each experiment, a 20 mL sample was taken from the starting solution, the permeate and the retentate, respectively, and evaporated to dryness at 40°C under reduced pressure. From the solid residue the amount of 7 -dehydrocholesterol (***DHC[%]***) and of compound of formula (V) (with R⁰=H)(***(V)[%]***) were determined by HPLC.

Furthermore, the ratio of ***DHC[%]*** and ***(V)[%]*** is indicated as ***(V)*/*DHC*** indicated in parts per million (ppm). Finally, for assessing the separation performance the reduction factor (***f_{red(V), perm}***) for compound of formula (V) between the starting solution and the permeate, and the increasing factor (***f_{inc(V),ret}** )* for compound of formula (V) between the starting solution and the retentate has been indicated in table 1.

### Experimental series 2: Two stage nanofiltration (according to figure 1b)

In the second series a starting solution as obtained above has been objected to a two-stage nanofiltration arranged in an experimental layout as above described in figure 1b.

In the first organic solvent nanofiltration unit the membrane *oNF-3* (MWCO = ca. 900 Dalton, Borsig Membrane Technology GmbH, Germany) having a surface area of 42 cm² has been used.

After 4 hours of nanofiltration of the starting solution the composition of the permeate and the retentate have been analysed (***7***) and indicated in table 2. The permeate has been used as starting solution for the second organic solvent nanofiltration unit using the membrane *oNF-2* (MWCO = ca. 350 Dalton, Borsig Membrane Technology GmbH, Germany) having a surface area of 42 cm². After 4 hours of nanofiltration the composition of the permeate and the retentate of the second organic solvent nanofiltration unit have been analysed (***8***) and indicated in table 2. The abbreviations used in table 2 are used accordingly to those of table 1. As a measure for the overall separation performance the ratio the reduction factor (***f_{Totalred(V), perm}***) for compound of formula (V) between the starting solution (introduced into the first organic solvent nanofiltration unit) and the permeate of the second organic solvent nanofiltration unit at the end of the two-stage nanofiltration has been indicated in table 2.

The comparison of the results shown in table 1 and 2 show that surprisingly particular the OSN membrane oNF-2 having a MWCO of about 350 Dalton shows an extraordinary good separation performance, whereas the OSN membranes having a higher MWCO (i.e. oNF-1 and oNF-3) show a very good, however lower that oNF-2, separation performance (***1, 2, 3, 6***) .

Furthermore, the results based on the OSN membrane oNF-2 show that the temperature have not a very high effect on the separation performance (***4, 5, 6***). Finally, table 2 shows that surprisingly the two-stage nanofiltration using two different OSN membranes show a significant better overall separation performance than the individual OSN nanofiltrations for the respective one-stage OSN nanofiltrations (***1***, ***7***; ***6***,***8***).

**Table 1 Results of nanofiltrations of DHC using different conditions.**

| | | | | | ***Starting solution*** | | | ***Permeate*** | | | ***Retentate*** | | | ***Permeate*** | ***Retentate*** |
|---|---|---|---|---|---|---|---|---|---|---|---|---|---|---|---|
| | ***Memb.*** | ***T [°C]*** | ***p [bara]*** | ***LF [ml*/*min]*** | ***DHC [%]*** | ***(V) [%]*** | ***(V)*/*DHC [ppm]*** | ***DHC [%]*** | ***(V) [%]*** | ***(V)*/*DHC [ppm]*** | ***DHC [%]*** | ***(V) [%]*** | ***(V)*/*DHC [ppm]*** | ***f_{red(V),perm}*** | ***f_{inc(V),ret}*** |
| ***1*** | *oNF-3* | 40 | 5 | 7.5 | 86.5 | 0.163 | *1884* | 86.7 | 0.0236 | *272* | 82.5 | 0.215 | *2606* | *7* | *1.32* |
| ***2*** | *oNF-1* | 40 | 10 | 4.5 | 86.9 | 0.127 | *1461* | 86.4 | 0.0151 | *175* | 88.7 | 0.152 | *1714* | *8* | *1.20* |
| ***3*** | *oNF-1* | 40 | 10 | 5.6 | 87.6 | 0.131 | *1495* | 89.7 | 0.0154 | *172* | 84.2 | 0.147 | *1746* | *9* | *1.12* |
| ***4*** | *oNF-2* | 20 | 10 | 5 | 83.5 | 0.165 | *1976* | 78.8 | 0.0106 | *135* | 76 | 0.166 | *2184* | *16* | *1.01* |
| ***5*** | *oNF-2* | 30 | 10 | 5 | 81.5 | 0.177 | *2175* | 89.4 | 0.0117 | *131* | 75.9 | 0.213 | *2806* | *15* | *1.20* |
| ***6*** | *oNF-2* | 40 | 10 | 5 | 87.4 | 0.174 | *1991* | 92.1 | 0.0074 | *80* | 81.4 | 0.176 | *2162* | *24* | *1.01* |

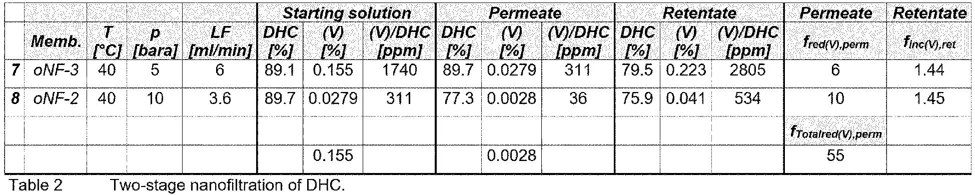

### Experimental series 3: One stage nanofiltration (according to figure 1a)

In the third series of examples, a starting solution of yield 25-hydroxy-7-dehydrocholesterol (formula (I) with R⁰=OH) (HyDHC) has been prepared as described above for DHC of experimental series 1 and 2.

For the following experiments a mixture of compounds of 25-hydoxy-dehydrocholesterol (HyDHC) and compound of formula (V) (with R⁰=OH) obtained by the above procedure has been dissolved in a solvent as indicated in table 3 to obtain a starting solution of 0.7 % HyDHC.

Said starting solution has been objected during 4 hours in a crossflow mode to a nanofiltration using the following membrane
oNF-2:MWCO = ca. 350 Dalton, Borsig Membrane Technology GmbH, Germany having a surface area of 42 cm² arranged in an experimental layout as above described in figure 1a.

The pressure (***p***), the temperature (**T**) and the loop feed (***LF***) used for the experiments have been indicated in table 3.

From each experiment, a 20 mL sample was taken from the starting solution, the permeate and the retentate, respectively, and evaporated to dryness at 40°C under reduced pressure. From the solid residue the amount of 25-hydroxy-7 - dehydrocholesterol (***HyDHC[%]***) and of compound of formula (V) (with R⁰=OH) (***(V)[%]***) were determined by HPLC.

Furthermore, the ratio of ***HyDHC[%]*** and ***(V)[%]*** is indicated as ***(V)*/*HyDHC*** indicated in parts per million (ppm). Finally, for assessing the separation performance the reduction factor (***f_{red(V), perm}***) for compound of formula (V) between the starting solution and the permeate, and the increasing factor (***f_{inc(V),ret}** )* for compound of formula (V) between the starting solution and the retentate has been indicated in table 3.

### Experimental series 4: Two stage nanofiltration (according to figure 1b)

In the fourth series a starting solution as obtained above has been objected to a two-stage nanofiltration arranged in an experimental layout as above described in figure 1b.

In the first organic solvent nanofiltration unit the membrane *oNF-2* (MWCO = ca. 350 Dalton, Borsig Membrane Technology GmbH, Germany) having a surface area of 42 cm² has been used.

After 4 hours of nanofiltration of the starting solution the composition of the permeate and the retentate have been analysed (***16***) and indicated in table 4. The permeate has been used as starting solution for the second organic solvent nanofiltration unit using the membrane *oNF-2* (MWCO=ca. 350 Dalton, Borsig Membrane Technology GmbH, Germany) having a surface area of 42 cm². After 4 hours of nanofiltration the composition of the permeate and the retentate of the second organic solvent nanofiltration unit have been analysed (***17***) and indicated in table 4. The abbreviations used in table 4 are used accordingly to those of table 3.

As a measure for the overall separation performance the ratio the reduction factor (***f_{Totalred(V), perm}***) for compound of formula (V) between the starting solution (introduced into the first organic solvent nanofiltration unit) and the permeate of the second organic solvent nanofiltration unit at the end of the two-stage nanofiltration has been indicated in table 4.

The comparison of the results shown in table 3 and 4 show that the OSN membrane oNF-2 having a MWCO of about 350 Dalton shows an extraordinary good separation performance.

Finally, table 4 shows that surprisingly the two-stage nanofiltration using twice the oNF-2 membrane show a significant better overall separation performance than the individual OSN nanofiltrations for the respective one-stage OSN nanofiltrations (***15, 16; 11, 13, 17***)*.*

**Table 3 Results of nanofiltrations of HyDHC using different conditions using the membrane oNF-2.* I/H=isopropanol/hexane.**

| | | | | | ***Starting solution*** | | | ***Permeate*** | | | ***Retentate*** | | | ***Permeate*** | ***Retentate*** |
|---|---|---|---|---|---|---|---|---|---|---|---|---|---|---|---|
| | ***T [°C]*** | ***p [bara]*** | ***LF [ml*/*min]*** | ***Solvent*** | ***HyDHC [%]*** | ***(V) [%]*** | ***(V)*/*HyDHC [ppm]*** | ***DHC [%]*** | ***(V) [%]*** | ***(V)*/*HyDHC [ppm]*** | ***DHC [%]*** | ***(V) [%]*** | ***(V)*/*HyDHC [ppm]*** | ***f_{red(V),perm}*** | ***f_{inc(V),ret}*** |
| ***9*** | 40 | 10 | 5.6 | THF | 90 | 0.179 | 1989 | 89.8 | 0.065 | 724 | 93.8 | 0.219 | 2335 | 3 | 1.22 |
| ***10*** | 40 | 30 | 2.3 | IPA | 82.3 | 0.343 | 4168 | 90.3 | 0.0207 | 229 | 92.3 | 0.463 | 5016 | 17 | 1.35 |
| ***11*** | 40 | 10 | 2.7 | I/H*(68/32) | 92.2 | 0.3719 | 4034 | 90.7 | 0.0116 | 128 | 90.9 | 0.378 | 4158 | 32 | 1.02 |
| ***12*** | 40 | 10 | 3.5 | I/H*(50/50) | 72.3 | 0.278 | 3845 | 82.3 | 0.009 | 109 | 86.6 | 0.369 | 4261 | 31 | 1.33 |
| ***13*** | 40 | 10 | 4.9 | I/H*(32/68) | 83.6 | 0.351 | 4199 | 92 | 0.0098 | 107 | 86.5 | 0.366 | 4231 | 36 | 1.04 |
| ***14*** | 60 | 10 | 7 | I/H*(32/68) | 92.9 | 0.39 | 4198 | 89.5 | 0.0153 | 171 | 91.2 | 0.397 | 4353 | 25 | 1.02 |
| ***15*** | 60 | 10 | 3.4 | I/H*(40/60) | 87.3 | 0.3139 | 3596 | 86.3 | 0.0137 | 159 | 83.2 | 0.319 | 3834 | 23 | 1.02 |

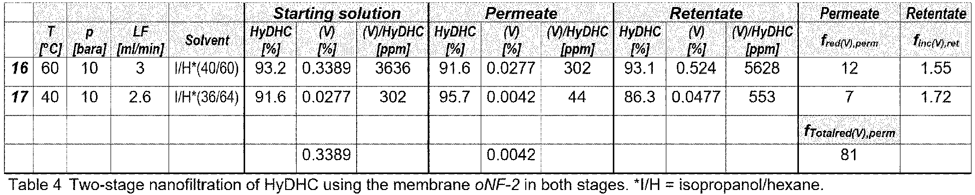

## Claims

1. Process of purification of a compound of the formula (I)
comprising the subsequent steps
a) providing a solution of a compound of formula (I) and a compound of formula (V) in a suitable solvent 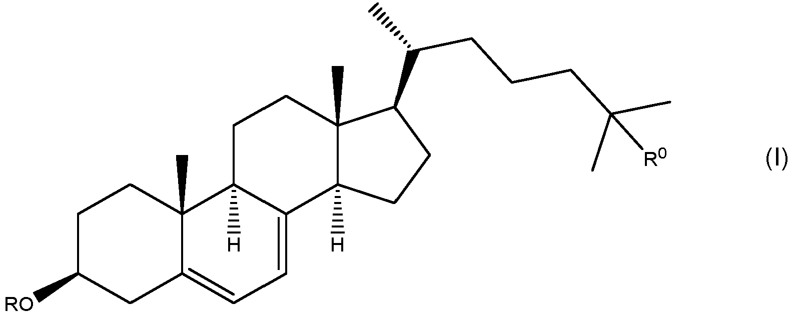 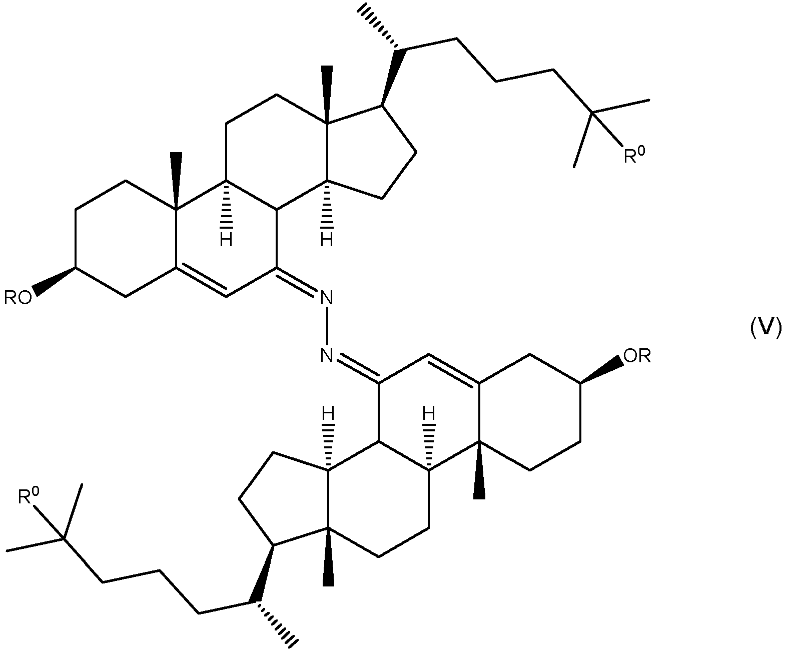
b) subjecting the solution of step a) by organic solvent nanofiltration using a membrane;
c) isolating the compound of formula (I) from the permeate obtained from step b)
wherein R represents H or an OH-protecting group; and R⁰ represents H or OH or OR' wherein R' represents an OH-protecting group.

2. The process according to claim 1 **characterized in that** the **characterized in that** that the membrane has a molecular weight cut-off of between 250 and 1000 Dalton, preferably of between 300 and 400 Dalton.

3. The process according to claim 1 or 2 **characterized in that** the membrane is a polymeric membrane, preferably a membrane based on silicone, most preferably a membrane which is a silicone-based composite.

4. The process according to any of the preceding claims **characterized in that** the pressure applied in the organic solvent nanofiltration of step b) is between 0.1 and 60 bar, preferably between 0.5 and 50 bar, more preferably 5 and 50 bar, even more preferably between 1 and 20 bar, most preferably between 8 and 15 bar.

5. The process according to any of the preceding claims **characterized in that** the organic solvent nanofiltration of step b) is performed at a temperature of between 15 and 60°C, preferably between 20 and 50°C.

6. The process according to any of the preceding claims **characterized in that** the solvent used in the organic solvent nanofiltration of step b) is a mixture of at least one alcohol and at least one C₅₋₁₀-alkane, preferably a mixture of a C₁₋₆-alcohol and a C₅₋₁₀-alkane, most preferably a mixture of hexane or heptane and methanol or ethanol, mostly preferred a mixture of hexane and methanol.

7. The process according to any of the preceding claims **characterized in that** in the case of R⁰ being different from H, the solvent used is either tetrahydrofuran or cyclopentylmethyl ether or isopropanol or a mixture of at least one alcohol and at least one C₅₋₁₀-alkane, preferably a mixture of a C₁₋₆-alcohol and a C₅₋₁₀-alkane, most preferably a mixture of hexane or heptane and isopropanol or methanol or ethanol, mostly preferred a mixture of hexane and isopropanol.

8. The process according to claim 6 or 7, **characterized in that** the weight ratio of alcohol and C₅₋₁₀-alkane is between 2:98 and 75:25, preferably between 10:90 and 65:35, most preferably between 15:85 and 50:50.

9. The process according to any of the preceding claims **characterized in that** the organic solvent nanofiltration is performed in a cross flow mode.

10. The process according to any of the preceding claims **characterized in that** the concentration of the compound of formula (I) in the solution at room temperature of step a) is between 0.1 and 20 % by weight, preferably between 0.5 and 10 % by weight, relative to the weight of the reaction mixture.

11. The process according to any of the preceding claims **characterized in that** the weight ratio of the compound of formula (V) to the compound of formula (I) in the solution of step a) is between 1:1'000'000 and 1:50, preferably between 1:900'000 and 1:200.

12. The process according to any of the preceding claims **characterized in that** the nanofiltration is a two-stage nanofiltration combining the use of two individual nanofiltration units having membranes of different molecular cut-off in sequence.

13. The use of organic solvent nanofiltration for the purification of a composition comprising compound of formula (I) 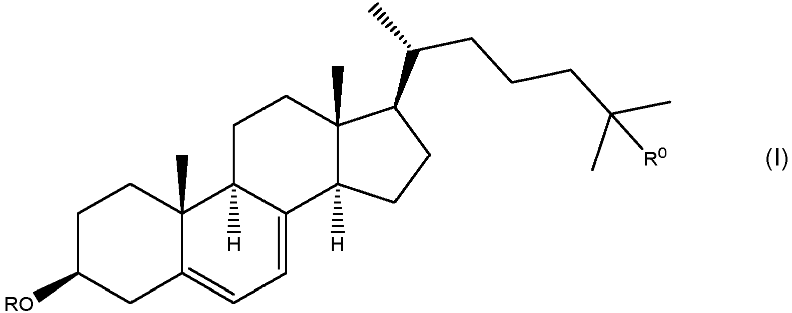
wherein R represents H or an OH-protecting group;
and R⁰ represents H or OH or OR' wherein R' represents an OH-protecting group.

## Patentansprüche

1. Verfahren zur Reinigung einer Verbindung der Formel (I),
bei dem man:
a) eine Lösung einer Verbindung der Formel (I) und einer Verbindung der Formel (V) in einem geeigneten Lösungsmittel bereitstellt 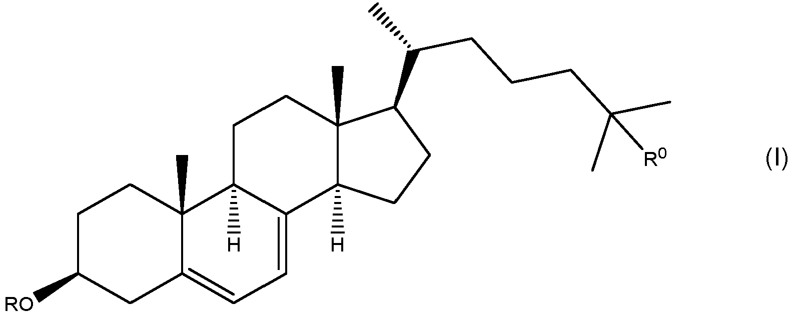 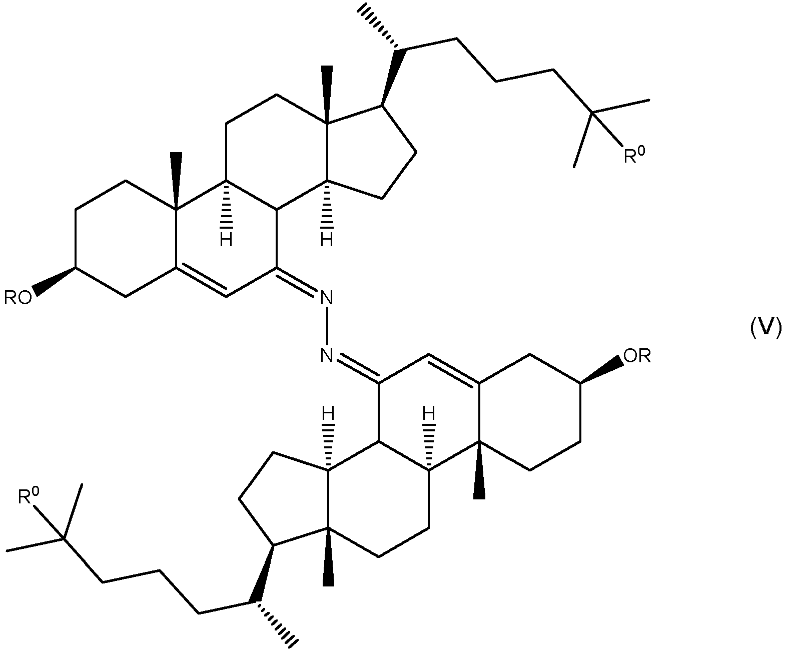
b) die Lösung aus Schritt a) einer organophilen Nanofiltration unter Verwendung einer Membran unterwirft;
c) die Verbindung der Formel (I) aus dem aus Schritt b) erhaltenen Permeat isoliert,
wobei R für H oder eine OH-Schutzgruppe steht und R⁰ für H oder OH oder OR' steht, wobei R' für eine OH-Schutzgruppe steht.

2. Verfahren nach Anspruch 1, **dadurch gekennzeichnet, dass** die Membran eine Molekulargewichtsausschlussgrenze zwischen 250 und 1000 Dalton, vorzugsweise zwischen 300 und 400 Dalton, aufweist.

3. Verfahren nach Anspruch 1 oder 2, **dadurch gekennzeichnet, dass** es sich bei der Membran um eine Polymermembran handelt, vorzugsweise eine Membran auf Basis von Silikon, ganz besonders bevorzugt eine Membran, bei der es sich um einen auf Silikon basierenden Verbund handelt.

4. Verfahren nach einem der vorhergehenden Ansprüche, **dadurch gekennzeichnet, dass** der bei der organophilen Nanofiltration von Schritt b) angewendete Druck zwischen 0,1 und 60 bar, vorzugsweise zwischen 0,5 und 50 bar, weiter bevorzugt 5 und 50 bar, noch weiter bevorzugt zwischen 1 und 20 bar, ganz besonders bevorzugt zwischen 8 und 15 bar, liegt.

5. Verfahren nach einem der vorhergehenden Ansprüche, **dadurch gekennzeichnet, dass** die organophile Nanofiltration von Schritt b) bei einer Temperatur zwischen 15 und 60 °C, vorzugsweise zwischen 20 und 50 °C, durchgeführt wird.

6. Verfahren nach einem der vorhergehenden Ansprüche, **dadurch gekennzeichnet, dass** es sich bei dem bei der organophilen Nanofiltration von Schritt b) verwendeten Lösungsmittel um eine Mischung von mindestens einem Alkohol und mindestens einem C₅₋₁₀-Alkan, vorzugsweise eine Mischung von mindestens einem C₁₋₆-Alkohol und einem C₅₋₁₀-Alkan, ganz besonders bevorzugt eine Mischung von Hexan oder Heptan uns Methanol oder Ethanol, höchst bevorzugt eine Mischung von Hexan und Methanol, handelt.

7. Verfahren nach einem der vorhergehenden Ansprüche, **dadurch gekennzeichnet, dass** es sich in dem Fall, dass R⁰ von H verschieden ist, bei dem Lösungsmittel entweder um Tetrahydrofuran oder Cyclopentylmethylether oder Isopropanol oder eine Mischung von mindestens einem Alkohol mit mindestens einem C₅₋₁₀-Alkan, vorzugsweise eine Mischung von einem C₁₋₆-Alkohol und einem C₅₋₁₀-Alkan, ganz besonders bevorzugt eine Mischung von Hexan oder Heptan und Isopropanol oder Methanol oder Ethanol, höchst bevorzugt eine Mischung von Hexan und Isopropanol, handelt.

8. Verfahren nach Anspruch 6 oder 7, **dadurch gekennzeichnet, dass** das Gewichtsverhältnis von Alkohol und C₅₋₁₀-Alkan zwischen 2:98 und 75:25, vorzugsweise zwischen 10:90 und 65:35, ganz besonders bevorzugt zwischen 15:85 und 50:50, liegt.

9. Verfahren nach einem der vorhergehenden Ansprüche, **dadurch gekennzeichnet, dass** die organophile Nanofiltration in Querstromfahrweise durchgeführt wird.

10. Verfahren nach einem der vorhergehenden Ansprüche, **dadurch gekennzeichnet, dass** die Konzentration der Verbindung der Formel (I) in der Lösung bei Raumtemperatur von Schritt a) zwischen 0,1 und 20 Gew.-%, vorzugsweise zwischen 0,5 und 10 Gew.-%, bezogen auf das Gewicht der Reaktionsmischung, liegt.

11. Verfahren nach einem der vorhergehenden Ansprüche, **dadurch gekennzeichnet, dass** das Gewichtsverhältnis von Verbindung der Formel (V) zu Verbindung der Formel (I) in der Lösung von Schritt a) zwischen 1:1.000.000 und 1:50, vorzugsweise zwischen 1:900.000 und 1:200, liegt.

12. Verfahren nach einem der vorhergehenden Ansprüche, **dadurch gekennzeichnet, dass** es sich bei der Nanofiltration um eine zweistufige Nanofiltration handelt, bei der die Verwendung von zwei individuellen Nanofiltrationseinheiten mit Membranen mit unterschiedlicher Molekulargewichtsausschlussgrenze hintereinander kombiniert wird.

13. Verwendung von organophiler Nanofiltration zur Reinigung einer Zusammensetzung, die eine Verbindung der Formel (I) umfasst, 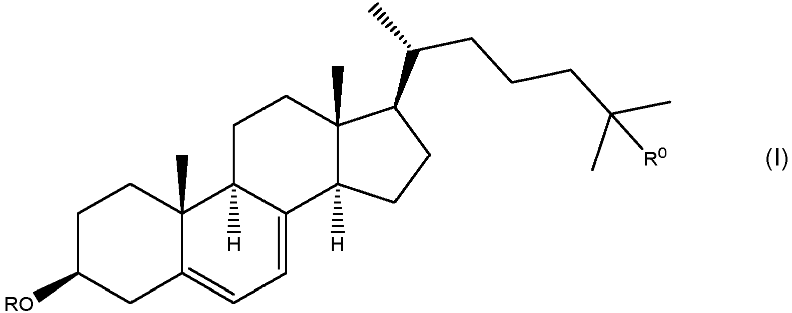
wobei R für H oder eine OH-Schutzgruppe steht
und R⁰ für H oder OH oder OR' steht, wobei R' für eine OH-Schutzgruppe steht.

## Revendications

1. Procédé de purification d'un composé de la formule (I)
comprenant les étapes subséquentes
a) fournir une solution d'un composé de formule (I) et d'un composé de formule (V) dans un solvant approprié 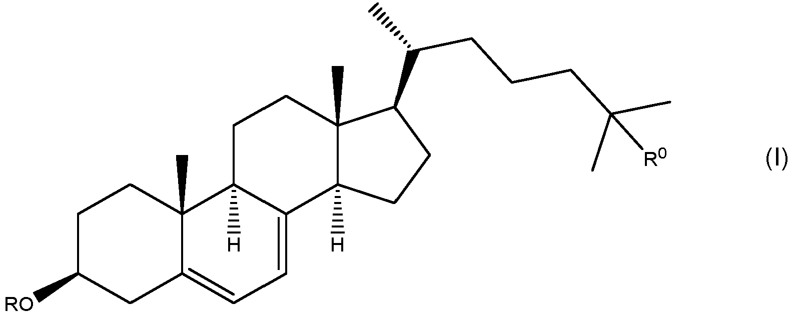 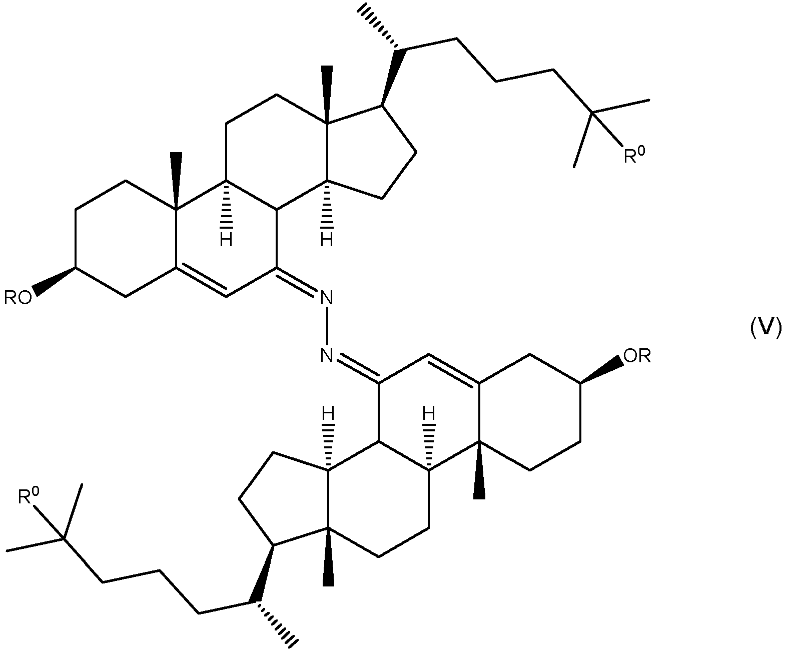
b) soumettre la solution de l'étape a) à une nanofiltration de solvant organique à l'aide d'une membrane ;
c) isoler le composé de formule (I) du perméat obtenu à l'étape b)
dans lequel R représente H ou un groupe protecteur de OH ; et R⁰ représente H ou OH ou OR', où R' représente un groupe protecteur de OH.

2. Procédé selon la revendication 1, **caractérisé en ce que** la membrane a un seuil de coupure de poids moléculaire compris entre 250 et 1 000 Daltons, de préférence entre 300 et 400 Daltons.

3. Procédé selon la revendication 1 ou 2, **caractérisé en ce que** la membrane est une membrane de polymère, de préférence une membrane à base de silicone, le plus préférablement une membrane qui est un composite à base de silicone.

4. Procédé selon l'une quelconque des revendications précédentes, **caractérisé en ce que** la pression appliquée dans la nanofiltration de solvant organique de l'étape b) est comprise entre 0,1 et 60 bars, de préférence entre 0,5 et 50 bars, plus préférablement entre 5 et 50 bars, encore plus préférablement entre 1 et 20 bars, le plus préférablement entre 8 et 15 bars.

5. Procédé selon l'une quelconque des revendications précédentes, **caractérisé en ce que** la nanofiltration de solvant organique de l'étape b) est effectuée à une température comprise entre 15 et 60 °C, de préférence entre 20 et 50 °C.

6. Procédé selon l'une quelconque des revendications précédentes, **caractérisé en ce que** le solvant utilisé dans la nanofiltration de solvant organique de l'étape b) est un mélange d'au moins un alcool et d'au moins un alcane en C₅₋₁₀, de préférence un mélange d'un alcool en C₁₋₆ et d'un alcane en C₅₋₁₀, le plus préférablement un mélange d'hexane ou d'heptane et de méthanol ou d'éthanol, le plus préférablement un mélange d'hexane et de méthanol.

7. Procédé selon l'une quelconque des revendications précédentes, **caractérisé en ce que** dans le cas où R⁰ est différent de H, le solvant utilisé est soit le tétrahydrofuranne, soit l'éther de cyclopentyle et de méthyle, soit l'isopropanol, soit un mélange d'au moins un alcool et d'au moins un alcane en C₅₋₁₀, de préférence un mélange d'un alcool en C₁₋₆ et d'un alcane en C₅₋₁₀, le plus préférablement un mélange d'hexane ou d'heptane et d'isopropanol ou de méthanol ou d'éthanol, le plus préférablement, un mélange d'hexane et d'isopropanol.

8. Procédé selon la revendication 6 ou 7, **caractérisé en ce que** le rapport pondéral de l'alcool et de l'alcane en C₅₋₁₀ est compris entre 2:98 et 75:25, de préférence entre 10:90 et 65:35, le plus préférablement entre 15:85 et 50:50.

9. Procédé selon l'une quelconque des revendications précédentes, **caractérisé en ce que** la nanofiltration de solvant organique est effectuée dans un mode à écoulement transversal.

10. Procédé selon l'une quelconque des revendications précédentes, **caractérisé en ce que** la concentration du composé de formule (I) dans la solution à température ambiante de l'étape a) est comprise entre 0,1 et 20 % en poids, de préférence entre 0,5 et 10 % en poids, par rapport au poids du mélange réactionnel.

11. Procédé selon l'une quelconque des revendications précédentes, **caractérisé en ce que** le rapport pondéral du composé de formule (V) sur le composé de formule (I) dans la solution de l'étape a) est compris entre 1:1 000 000 et 1:50, de préférence entre 1:900 000 et 1:200.

12. Procédé selon l'une quelconque des revendications précédentes, **caractérisé en ce que** la nanofiltration est une nanofiltration en deux étapes combinant l'utilisation de deux unités de nanofiltration individuelles ayant des membranes de seuil de coupure moléculaire différente en séquence.

13. Utilisation d'une nanofiltration de solvant organique pour la purification d'une composition comprenant un composé de formule (I) 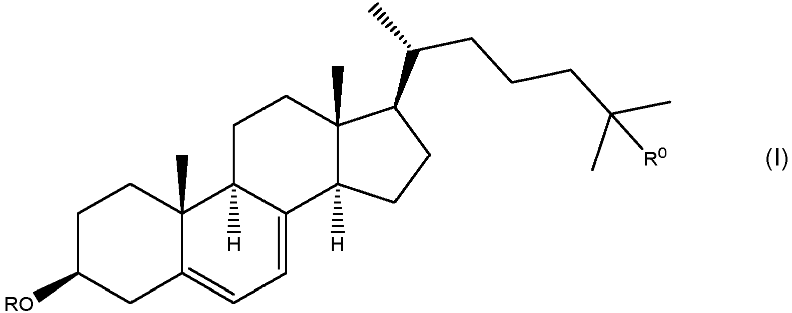
dans laquelle R représente H ou un groupe protecteur de OH ;
et R⁰ représente H ou OH ou OR', où R' représente un groupe protecteur de OH.
